# DEMANDE DE BREVET EUROPEEN

(11) **EP 1 214 918 A1**
(43) Date de publication de la demande: **19.06.2002**
(21) Numéro de dépôt: 01403110.8
(22) Date de dépôt: 04.12.2001
(51) Int. Cl.: A61F 2/44

(54) **Prothèse intervertebrale**

(30) Priorité: 11.12.2000 FR 0016054
(71) Demandeur: Bouvet, Jean-Claude, F-91590 Mondeville (FR)
(72) Inventeur: Bouvet, Jean-Claude, F-91590 Mondeville (FR)
(74) Mandataire: Flavenot, Bernard

(57) **Abrégé**

La présente invention concerne les prothèses intervertébrales.

La prothèse selon l'invention se caractérise essentiellement par le fait qu'elle comporte deux plaques supports 1, 2 parallèles et reliée entre elles de façon qu'elles soient aptes à pivoter l'une par rapport au moyen d'une lame élastique 6 en étoile à au moins quatre branches 9 et deux bordures 11, 12 respectivement solidaires des faces en regard 26, 27 respectivement des première et seconde plaques supports 1, 2, la somme des hauteurs de ces deux bordures 11, 12 étant inférieure à la distance séparant les deux faces en regard 26, 27 quand les deux plaques supports 1, 2 sont parallèles, pour définir entre ces deux bordures un espace de débattement limite 10 pour la rotation des deux plaques supports l'une par rapport à l'autre autour d'au moins un deuxième axe perpendiculaire au premier axe 5.

## Description

La présente invention concerne les prothèses destinées à restaurer une articulation entre deux os, qui trouvent une application particulièrement avantageuse comme prothèses intervertébrales.

On connaît bon nombre de prothèses implantables dans le corps humain ou analogue pour restaurer des articulations défectueuses, par exemple à la suite d'accidents ou de maladies.

Ces prothèses articulaires sont pour la plupart essentiellement constituées de deux parties respectivement solidarisées aux deux os de l'articulation et montées mobiles l'une par rapport à l'autre, par exemple à la manière d'un mouvement rotulien.

Cependant, ce type de prothèse peut à la longue présenter un inconvénient. En effet, lorsque les deux parties de prothèse se déplacent l'une par rapport à l'autre, il se produit entre elles des frottements qui génèrent des particules d'usure solides du matériau ou des matériaux qui la composent. De façon schématique, ces particules infiniment petites sont considérées, par le corps vivant dans lequel est implantée la prothèse, comme des corps étrangers dont l'élimination échoit aux cellules dites "macrophages". Or, la nature des matériaux constituant ces particules d'usure ne leur permet pas d'être "digérées" par les cellules macrophages qui s'accumulent alors de plus en plus nombreuses autour d'elles, c'est-à-dire entre la prothèse et les tissus vivants. Ces agrégats que l'organisme vivant n'arrive pas à éliminer ont tendance à désolidariser la prothèse et les os dans lesquels elle a été implantée et sont donc fortement nuisibles au maintien durable d'une telle prothèse.

Pour tenter de pallier ces inconvénients, les techniciens ont réalisé des prothèses intervertébrales tendant à éliminer le plus possible les effets de friction entre les parties mobiles les unes sur les autres. C'est ainsi qu'il a été réalisé des prothèses pour restaurer l'articulation entre deux vertèbres, schématiquement constituées de deux plaques supports et de coussins disposés entre ces deux plaques, les coussins étant réalisés en un matériau élastique permettant de restituer les mouvements relatifs des deux vertèbres.

Pour sa part, le Demandeur a réalisé une prothèse comportant une première plaque support, des premiers moyens pour solidariser la première plaque support à un premier os, une seconde plaque support, des seconds moyens pour solidariser la seconde plaque support au second os, des moyens pour relier les deux plaques supports de façon qu'elles soient sensiblement parallèles en délimitant entre elles un espace et qu'elles pivotent l'une par rapport à l'autre au moins et principalement autour d'un axe sensiblement perpendiculaire à leur plan général, les moyens pour relier les deux plaques supports étant constitués d'une lame élastique formant sensiblement une étoile à au moins quatre branches, cette lame élastique ayant une largeur nettement plus grande que son épaisseur mais inférieure à la distance séparant les deux plaques supports, la lame élastique étant en outre située dans l'espace défini entre les deux plaques supports de façon que sa largeur soit sensiblement parallèle à l'axe et étant réalisée en un matériau présentant une certaine élasticité, des troisièmes moyens pour solidariser les deux extrémités libres de deux branches opposées de l'étoile avec la première plaque support, et des quatrièmes moyens pour solidariser les deux extrémités libres des deux autres branches opposées de l'étoile avec la seconde plaque support.

La prothèse définie ci-dessus donne satisfaction. Cependant, la présente invention a pour but de la perfectionner en tendant notamment de la rendre encore plus fiable.

Plus précisément, la présente invention a pour objet une prothèse intervertébrale comportant une première et une seconde plaques supports, des moyens pour relier les deux plaques supports de façon qu'elles soient sensiblement parallèles en délimitant entre elles un espace et aptes à pivoter l'une par rapport à l'autre au moins et principalement autour d'un premier axe sensiblement perpendiculaire à leur plan général, ces moyens pour relier les deux plaques supports étant constitués par une lame élastique formant sensiblement une étoile à au moins quatre branches, cette lame élastique ayant une largeur nettement plus grande que son épaisseur mais inférieure à la distance séparant les deux dites plaques supports, ladite lame élastique étant en outre située dans ledit espace de façon que sa largeur soit sensiblement parallèle audit premier axe, des moyens pour solidariser les deux extrémités libres de deux branches opposées de l'étoile avec ladite première plaque support et des moyens pour solidariser les deux extrémités libres des deux autres branches opposées de l'étoile avec la seconde plaque support, caractérisée par le fait qu'elle comporte en outre deux bordures respectivement solidaires des faces en regard respectivement des première et seconde plaques supports de façon sensiblement continue et sur tout leur pourtour, chaque bordure étant sensiblement perpendiculaire au plan de la face à laquelle elle est solidarisée, la somme des hauteurs de ces deux bordures étant inférieure à la distance séparant les deux faces en regard quand les deux plaques supports sont parallèles, pour définir entre ces deux bordures un espace de débattement limite pour la rotation des deux plaques supports l'une par rapport à l'autre autour d'au moins un deuxième axe perpendiculaire audit premier axe.

D'autres caractéristiques et avantages de la présente invention apparaîtront au cours de la description suivante donnée en regard des dessins annexés à titre illustratif mais nullement limitatif, dans lesquels :
Les figures 1 A, B et C représentent respectivement une vue de côté en coupe (fig. 1A) référencée a-a sur la figure 1B, une vue de face (fig. 1B) et une vue de dessus en coupe (fig. 1C) référencée c-c sur la figure 1B, d'un mode de réalisation préféré d'un des éléments constitutifs de la prothèse intervertébrale selon l'invention,
La figure 2 représente de façon schématique, vue de côté suivant la flèche "F" sur la figure 1B et à échelle dilatée non linéaire, une prothèse intervertébrale selon l'invention comportant deux éléments comme illustrés sur la figure 1, et
Les figures 3 et 4 représentent de façon schématique et partielle, deux vues en coupe perpendiculaires d'un autre mode de réalisation de la prothèse intervertébrale selon l'invention, en accord avec les représentations des figures 1 et 2.

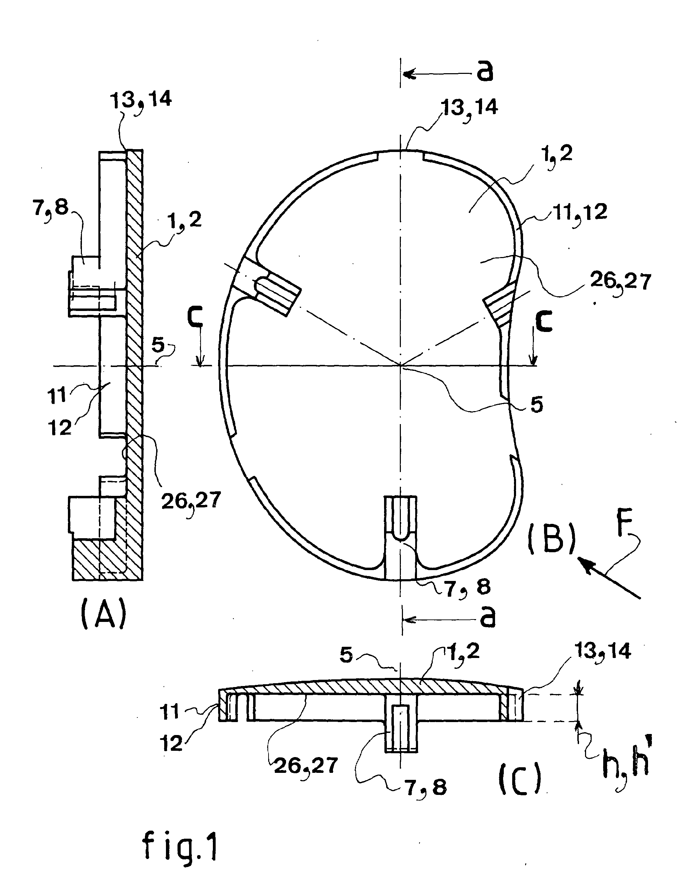

Le Demandeur tient à préciser que les figures 1 à 4 représentent de façon schématique plusieurs modes de réalisation de l'objet de l'invention et qu'il peut exister d'autres modes de réalisation qui répondent à la définition de cette invention.

Il précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si le mode de réalisation de l'objet de l'invention tel qu'illustré comporte plusieurs éléments de fonction identique et que si, dans la description, il n'est pas spécifié que l'objet de l'invention doit obligatoirement comporter un nombre particulier de ces éléments, il pourra être défini comme comportant "au moins un" de ces éléments.

De plus, dans le but de faciliter la compréhension de la description suivante, les mêmes références désignent les mêmes éléments, quelle que soit la figure sur laquelle elles apparaissent et quelle que soit la forme de représentation de ces éléments.

Il précise aussi que, la présente invention concerne un perfectionnement à la prothèse telle que décrite dans le Brevet Français 2 761 878 qui définit l'art antérieur le plus proche de cette invention.

Ceci ayant été précisé, la présente invention est relative à une prothèse permettant de restaurer une articulation entre deux os, notamment deux vertèbres.

Par référence à l'ensemble des figures, la prothèse intervertébrale comporte une première et une seconde plaques supports 1, 2, des moyens 3 pour relier ces deux plaques supports de façon qu'elles soient sensiblement parallèles en délimitant entre elles un espace 4 et aptes à pivoter l'une par rapport à l'autre au moins et principalement autour d'un premier axe 5 sensiblement perpendiculaire à leur plan général.

Ces moyens 3 pour relier les deux plaques supports sont constitués par une lame élastique 6 en forme sensiblement d'une étoile à au moins quatre branches 9, avantageusement six branches comme illustré sur les figures. Cette lame élastique a une largeur nettement plus grande que son épaisseur mais inférieure à la distance séparant les deux plaques supports. Elle est en outre située dans l'espace 4 de façon que sa largeur soit sensiblement parallèle au premier axe 5.

La prothèse comporte en outre, dans le cas où la lame élastique en étoile comporte quatre branches, des moyens 7 pour solidariser les deux extrémités libres de deux branches opposées de l'étoile avec la première plaque support 1 et des moyens 8 pour solidariser les deux extrémités libres des deux autres branches opposées de l'étoile avec la seconde plaque support 2. Dans le cas, comme illustré sur les figures, où la lame élastique en étoile comporte six branches, l'extrémité libre de une branche sur deux est solidarisée avec une plaque support, l'extrémité libre des trois autres branches étant solidarisée avec l'autre plaque support. Ces moyens 7, 8 sont par exemple constitués, comme plus particulièrement visible sur la figure 1, par des encoches réalisées dans chaque plaque support, dans lesquelles s'enfichent en force les extrémités libres des branches de l'étoile.

Selon une caractéristique essentielle de l'invention, le prothèse comporte en outre deux bordures 11, 12 respectivement solidaires des faces en regard 26, 27 respectivement des première et seconde plaques supports 1, 2, de façon sensiblement continue et sur tout leur pourtour 13, 14. Chaque bordure est sensiblement perpendiculaire au plan de la face à laquelle elle est solidarisée et la somme des hauteurs h, h' de ces deux bordures 11, 12 est inférieure à la distance séparant les deux faces en regard 26, 27 quand les deux plaques supports 1, 2 sont parallèles, pour définir entre ces deux bordures un espace de débattement limite 10 pour la rotation des deux plaques supports l'une par rapport à l'autre autour d'au moins un deuxième axe perpendiculaire au premier axe 5.

Dans une réalisation avantageuse, les deux ensembles comportant chacun une plaque support 1, 2 et une bordure 11, 12 ont des formes identiques et, dans ce cas, les deux hauteurs h et h' sont égales.

Outre qu'elles permettent de limiter le débattement angulaire des deux plaques supports comme mentionné ci-avant, les deux bordures 11, 12 permettent d'éviter que des fibroses s'introduisent dans l'espace 4 défini ci-avant et viennent perturber le fonctionnement de la prothèse.

Cependant, comme dans le cas de la prothèse de l'art antérieur défini par le Brevet Français identifié ci-dessus, la prothèse peut comporter en outre une gaine enveloppant l'ensemble des plaques supports 1, 2 et des deux bordures 11, 12.

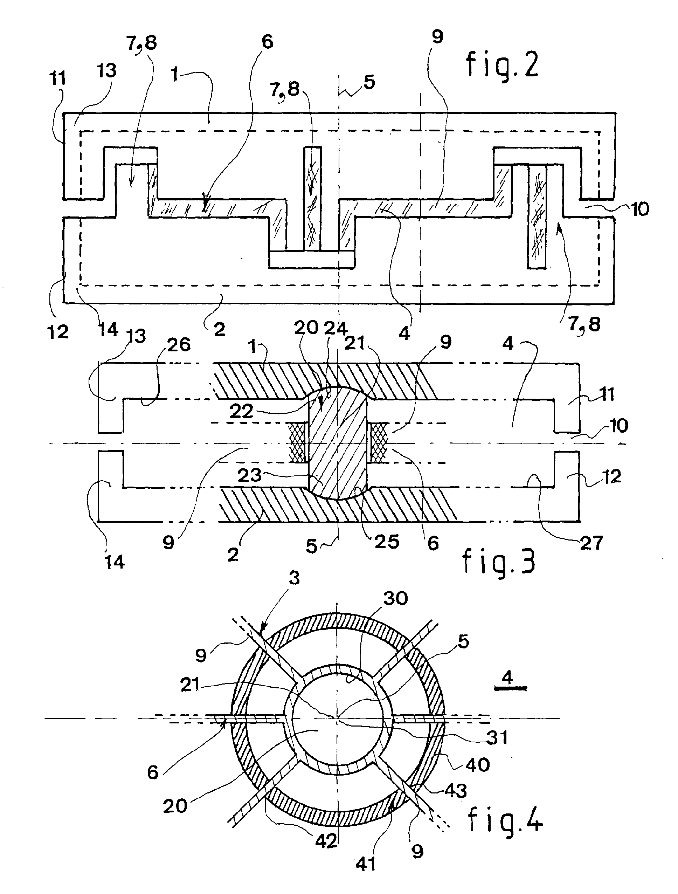

Dans une réalisation possible dans certains cas d'application de cette prothèse selon l'invention, elle peut comporter en outre, comme illustré sur les figures 3 et 4, un pivot 20 dont l'axe 21 est confondu avec le premier axe 5 et dont au moins l'une des deux extrémités 22, 23, et avantageusement les deux comme illustré, est montée rotative sur l'une des deux plaques supports 1, 2.

Dans le mode de réalisation illustré, les deux extrémités 22, 23 du pivot 20 sont réalisées en forme de portion de sphère et sont montées rotatives dans des cavités correspondantes 24, 25 réalisées respectivement dans les deux faces en regard 26, 27 des deux plaques supports.

Il peut dans ce cas être avantageux que la lame élastique 6 comporte, en son centre, une traversée 30 d'axe 31 et de section sensiblement complémentaire de celle du pivot 20 définie suivant un plan perpendiculaire à son axe 21, le pivot étant alors situé dans cette traversée de façon que son axe soit sensiblement confondu avec l'axe 31 de la traversée.

Selon une autre réalisation, la prothèse peut comporter au moins un capot 40 en forme de cuvette, dont le bord 41 comporte des encoches 42, 43, ... aptes à venir se placer sur les branches 9 de la lame élastique en étoile 6, afin d'obtenir une rigidité déterminée de cette lame élastique. De façon avantageuse, le praticien qui doit implanter une telle prothèse aura à sa disposition une pluralité de ces capots 40 de différents diamètres pour pouvoir ajuster la rigidité de la lame élastique, en sachant que cette rigidité est d'autant plus importante que le diamètre du capot est grand.

La prothèse peut avantageusement comporter en outre un matériau de remplissage de l'espace 4 défini entre les deux plaques supports 1, 2 et les deux bordures 11, 12, par exemple un élastomère biocompatible dont l'élasticité est supérieure à celle de la lame élastique 6.

Elle peut aussi comporter en outre des moyens pour adapter la rigidité d'au moins une des branches 9 de la lame élastique en étoile 6. Ces moyens peuvent être constitués de différentes façons, par exemple par des orifices réalisés dans la branche, des surépaisseurs de la branche, des cavaliers disposés sur la branche, etc.

## Revendications

1. Prothèse intervertébrale comportant une première et une seconde plaques supports (1, 2), des moyens (3) pour relier les deux plaques supports (1, 2) de façon qu'elles soient sensiblement parallèles en délimitant entre elles un espace (4) et aptes à pivoter l'une par rapport à l'autre au moins et principalement autour d'un premier axe (5) sensiblement perpendiculaire à leur plan général, ces moyens (3) pour relier les deux plaques supports étant constitués par une lame élastique (6) formant sensiblement une étoile à au moins quatre branches (9), cette lame élastique ayant une largeur nettement plus grande que son épaisseur mais inférieure à la distance séparant les deux dites plaques supports, ladite lame élastique étant en outre située dans ledit espace (4) de façon que sa largeur soit sensiblement parallèle audit premier axe (5), des moyens (7) pour solidariser les deux extrémités libres de deux branches opposées de l'étoile avec ladite première plaque support (1) et des moyens (8) pour solidariser les deux extrémités libres des deux autres branches opposées de l'étoile avec la seconde plaque support (2), **caractérisée par le fait qu'**elle comporte en outre deux bordures (11, 12) respectivement solidaires des faces en regard (26, 27) respectivement des première et seconde plaques supports (1, 2), de façon sensiblement continue et sur tout leur pourtour (13, 14), chaque bordure étant sensiblement perpendiculaire au plan de la face à laquelle elle est solidarisée, la somme des hauteurs de ces deux bordures (11, 12) étant inférieure à la distance séparant les deux faces en regard (26, 27) quand les deux plaques supports (1, 2) sont parallèles, pour définir entre ces deux bordures un espace de débattement limite (10) pour la rotation des deux plaques supports l'une par rapport à l'autre autour d'au moins un deuxième axe perpendiculaire audit premier axe (5).

2. Prothèse selon la revendication 1, **caractérisée par le fait que** ladite lame élastique (6) forme une étoile à au moins six branches.

3. Prothèse selon la revendication 2, **caractérisée par le fait que** les deux ensembles comportant chacun une plaque support (1, 2) et une bordure (11, 12) ont des formes identiques.

4. Prothèse selon l'une des revendications 1 à 3, **caractérisée par le fait qu'**elle comporte en outre un pivot (20), l'axe (21) dudit pivot étant confondu avec ledit premier axe (5), au moins l'une des deux extrémités (22, 23) dudit pivot (20) étant montée rotative sur l'une des deux plaques supports (1, 2).

5. Prothèse selon la revendication 4, **caractérisée par le fait que** les deux extrémités (22, 23) dudit pivot (20) sont en forme de portion de sphère et sont montées rotatives dans des cavités correspondantes (24, 25) réalisées respectivement dans les deux faces en regard (26, 27).

6. Prothèse selon l'une des revendications 4 et 5, **caractérisée par le fait que** ladite lame élastique (6) comporte en son centre une traversée (30), la section de cette traversée (30) étant sensiblement complémentaire de la section dudit pivot (20) définie suivant un plan perpendiculaire à son axe (21), ledit pivot étant situé dans cette dite traversée.

7. Prothèse selon l'une des revendications 1 à 6, **caractérisée par le fait qu'**elle comporte en outre au moins un capot (40) en forme de cuvette, dont le bord (41) comporte des encoches (42, 43, ...) aptes à venir se placer sur les branches (9) de la lame élastique (6) en étoile, afin d'obtenir une rigidité déterminée de ladite lame élastique (6).

8. Prothèse selon l'une des revendications 1 à 7, **caractérisée par le fait qu'**elle comporte en outre un matériau de remplissage de l'espace (4) défini entre les deux plaques supports (1, 2) et les deux bordures (11, 12).

9. Prothèse selon la revendication 8, **caractérisée par le fait que** ledit matériau est un élastomère biocompatible dont l'élasticité est supérieure à celle de la lame élastique (6)

10. Prothèse selon l'une des revendications 1 à 9, **caractérisée par le fait qu'**elle comporte en outre des moyens pour adapter la rigidité d'au moins une des branches (9) de ladite lame élastique (6) en étoile.
